(19) Europäisches Patentamt
European Patent Office
Office européen des brevets

(11) Veröffentlichungsnummer: **0 024 258**
**A1**

(12) # EUROPÄISCHE PATENTANMELDUNG

(21) Anmeldenummer: **80810247.9**

(22) Anmeldetag: **08.08.80**

(51) Int. Cl.³: **A 61 K 31/135**
//C07C87/458

(30) Priorität: **14.08.79 CH 7429/79**

(43) Veröffentlichungstag der Anmeldung:
**25.02.81 Patentblatt 81/8**

(84) Benannte Vertragsstaaten:
**BE CH FR GB IT LI NL SE**

(71) Anmelder: **CIBA-GEIGY AG**
**Patentabteilung Postfach**
**CH-4002 Basel(CH)**

(72) Erfinder: **Schmidt-Ruppin, Karl Heinz, Dr.**
**Im Baumgarten 5**
**CH-4144 Arlesheim(CH)**

(72) Erfinder: **Lukas, Bohumir, Dr.**
**Florastrasse 18/4**
**CH-4057 Basel(CH)**

(72) Erfinder: **Wiesendanger, Walter**
**Steingrubenweg 12**
**CH-4142 Münchenstein(CH)**

(54) **Pharmazeutische Zusammensetzungen zur Behandlung von Herpes-Infektionen.**

(57) Verfahren zur Behandlung von Herpes-Infektionen, durch Verabreichung einer Verbindung der Formel

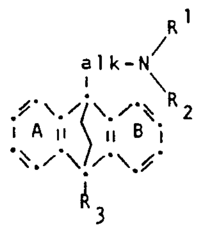

1

worin alk für Alkylen mit 1-4 Kohlenstoffatomen steht, $R_1$ und $R_2$ unabhängig voneinander Wasserstoff, Alkyl mit 1-4 Kohlenstoffatomen bedeuten oder zusammen für einen Alkylenrest mit 4 bis 6 Kohlenstoffatomen stehen, $R_3$ Wasserstoff, Methyl oder Chlor bedeuten, und die Ringe A und B unabhängig voneinander unsubstituiert oder durch Chlor substituiert sein können, oder ihren pharmazeutisch annehmbaren Säureadditionssalze, sowie die zur Anwendung in diesem Verfahren geeigneten pharmazeutischen Zusammensetzungen.

EP 0 024 258 A1

Croydon Printing Company Ltd.

BEZEICHNUNG GEÄNDERT
siehe Titelseite



CIBA-GEIGY AG
Basel (Schweiz)

4-12473/+

Verfahren zur Behebung oder Linderung von Herpes-Infektionen und hierzu geeignete pharmazeutische Zusammensetzungen

Die vorliegende Erfindung betrifft ein Verfahren zur Behebung oder Linderung von Herpes-Infektionen, sowie die zur Anwendung in diesem Verfahren geeigneten pharmazeutischen Zusammensetzungen.

Es wurde überraschenderweise gefunden, dass Verbindungen der Formel I

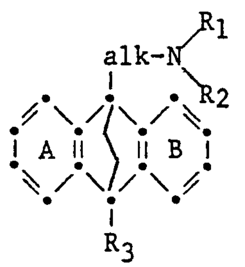

I

worin alk für Alkylen mit 1-4 Kohlenstoffatomen steht, $R_1$ und $R_2$ unabhängig voneinander Wasserstoff, Alkyl mit 1-4 Kohlenstoffatomen bedeuten oder zusammen für einen Alkylenrest mit 4 bis 6 Kohlenstoffatomen stehen, $R_3$ Wasserstoff, Methyl oder Chlor bedeuten, und die Ringe A und B unabhängig voneinander unsubstituiert oder durch Chlor substituiert sein können, und ihre pharmazeutisch annehmbaren Säureadditionssalze an Versuchstieren bei experimentellen mucocutanen und cutanen Herpesinfektionen des Meerschweinchens sowie auch bei experimenteller Herpesencephalitis (Gehirnentzündung) der Maus hoch wirksam sind.

Die Verbindungen der Formel I und ihre Säureadditionssalze sind in der US-PS 3 399 201 beschrieben. Gemäss dieser Patentschrift wirken solche Verbindungen und ihre pharmazeutisch annehmbaren Säureadditionssalze auf das zentrale Nervensystem von Säugetieren und können als Wirkstoffe für Arzneimittel verwendet werden. Aus diesen Angaben konnte in keiner Weise geschlossen werden, dass die Verbindungen der Formel I und ihre pharmazeutisch annehmbaren Säureadditionssalze zur

Bekämpfung von Herpes-Infektionen geeignet sein würden.

In den Verbindungen der Formel I steht alk insbesondere für Trimethylen und vor allem für Methylen. $R_1$ und $R_2$ stehen insbesondere für Aethyl und Propyl und ganz besonders für Wasserstoff und Methyl, wobei vor allem $R_1$ Wasserstoff und $R_2$ gleichzeitig Methyl bedeutet. Die Ringe A und B können unabhängig voneinander einfach oder mehrfach substituiert sein, sie sind aber vorzugsweise einfach substituiert und zwar steht Chlor insbesondere in 2- oder 3-Stellung. Besonders bevorzugt sind aber Verbindungen, in denen die Ringe A und B unsubstituiert sind. Eine als Wirkstoff bevorzugt verwendete Verbindung ist 9-[3-(Methylamino)-propyl]-9,10-dihydro-9,10-ethanoanthracen und ganz besonders 9-[(Methylamino)-methyl]-9,10-dihydro-9,10-ethano-anthracen.

Als pharmazeutisch annehmbare Salze der Verbindungen der Formel I können beispielsweise das Hydrochlorid, Hydrobromid, Phosphat, Ethansulfonat, 2-Hydroxyethansulfonat, Acetat, Lactat, Malonat, Succinat, Fumarat, Maleinat, Malat, Tartrat, Citrat, Benzoat, Salicylat, Phenylacetat, Mandelat oder Embonat und vor allem das Methansulfonat verwendet werden.

Die antiherpetische Wirksamkeit von erfindungsgemässen pharmazeutischen Präparaten kann tierexperimentell z.B. an dem durch Infektion mit HVH 2/Angelotti verursachten Herpes genitalis des Meerschweinchens bei 72 Stunden nach Infektion (Stadium deutlicher Symptome) beginnender Behandlung festgestellt werden, wobei auf die von B. Lukas et al., Arch. Ges. Virusforsch. 44, 153-155 (1974) und 49, 1-11 (1975) beschriebene Methodik hingewiesen wird. Beispielsweise geht die starke Wirksamkeit von 9-[(Methylamino)-methyl]-9,10-dihydro-9,10-ethanoanthracen-hyrochlorid bei oraler Verabreichung aus der nachstehenden Tabelle 1a) hervor.

Das Auftreten von Rezidiven war nach dieser Behandlung stark vermindert (vgl. Tabelle 1b).

Die intravaginale Applikation von pro Tag zweimal 0,1 ml eines 0,1%-igen Gels von 9-[(Methylamino)-methyl]-9,10-dihydro-9,10-ethanoanthracen-hydrochlorid bewirkte ebenfalls eine rasche Regression der lokalen Symptome der Herpes genitalis bis zum völligen Verschwinden derselben.

Bei der durch Infektion mit HVH 1/Tup erzeugten Dermatitis herpetica beschleunigte die Behandlung ab 72 Stunden nach Infektion mit 5,0, 1,0 und 0,2 mg/kg p.o. (2 x täglich 5 Tage lang) die Rückbildung der Hautläsionen, wie aus Tabelle 2 ersichtlich ist.

Bei ascendierender, von Paralyse und Exitus gefolgter vaginaler Infektion des Meerschweinchens mit 3 x $10^3$ infektiösen Einheiten (PFU = Plaque forming units) des besonders neurotropen Stammes HVH 2/Alabama war 9-[(Methylamino)-methyl]-9,10-dihydro-9,10-ethanoanthracen-hydrochlorid ebenfalls wirksam (Tabelle 3).

Die prophylaktische und frühtherapeutische perorale Behandlung erhöhte die Ueberlebensrate von Mäusen nach cerebraler Infektion mit 5 x $LD_{90}$ von HVH 1/Tup (Tabelle 4). Aus den Prozentzahlen der überlebenden Kontrolltiere geht hervor, dass die Infektion nicht in allen Versuchsgruppen gleich stark war. Gleichwohl ist aus den Resultaten zu erkennen, dass bei vor der Infektion einsetzender Behandlung die höchste Wirkstoffdosierung gleich oder stärker wirkte als niedrigere, während bei späterem Behandlungsbeginn gemäss Versuchsgruppen 3 und 4 die niedrigste Dosierung relativ am stärksten wirkte.

Tabelle 1a)

Wirksamkeit von 9-[(Methylamino)-methyl]-9,10-dihydro-9,10-ethanoanthracen-hydrochlorid in einem Blindversuch an Meerschweinchen mit durch Infektion mit HVH2/Ang. verursachtem Herpes genitalis

| Gruppe | Dosis mg/kg p.o. | N | Wirkung auf lokale Symptome | | | | | | | | | Tiere | |
|---|---|---|---|---|---|---|---|---|---|---|---|---|---|
| | | | Tiere mit mindestens 66% Regression | | | | Symptomfreie Tiere | | | | | | |
| | | | Tag 4 | 7 | 9 | 11 | 9 | 11 | 14 | 18 | 25 | tot | gelähmt |
| 1 | 5,0 | 20 | 2 | 7** | 11** | 14** | 5* | 8**x | 9**x | 12* | 15* | 0 | 2 |
| 2 | 1,0 | 20 | 0 | 11** | 15** | 16** | 10** | 16** | 16** | 17** | 20** | 0 | 0 |
| 3 | 0,2 | 20 | 1 | 10** | 14** | 15** | 8** | 9**x | 12** | 15** | 17** | 0 | 0 |
| 4 | Placebo | 20 | 0 | 0 | 0 | 1 | 0 | 0 | 0 | 3 | 6 | 0 | 0 |

Behandlung: zweimal täglich während 5 Tagen, beginnend am Tag 0, 3 Tage nach der Infektion.

* $P<0.05$ } verschieden von Placebo } Fisher Exact Test
** $P<0.01$ }
x $P<0.05$ verschieden von Gruppe 2
N = Anzahl der Tier

Tabelle 1b)

Das Auftreten von Rezidiven bei Herpes genitalis in Meerschweinchen nach peroraler Behandlung mit 9-[(Methylamino)-methyl]-9,10-dihydro-9,10-ethanoanthracen-hydrochlorid

| Wirkstoff und Dosierung | Anzahl der Tiere [a] mit Rezidiven nach Tagen 28 | 60 | 90 | total | Anzahl der Tiere mit Rezidiven von ..Tagen Dauer 7 | 14 | 21 | 28 Tage |
|---|---|---|---|---|---|---|---|---|
| 3-[(Methylamino)-methyl]-9,10-dihydro-9,10-ethanoanthracen-hydrochlorid | | | | | | | | |
| 5,0 mg/kg | 1 | | | 1 | | | 1 | |
| 1,0 mg/kg | 0 | 0 | 0 | 0 | | | | |
| 0,2 mg/kg | 1 | | 1 | 2 | 1 | 1 | | |
| Placebo | 4 | 4 | 3 | 11[b] | 6 | 4 | 2 | 1 |

N = 20 (siehe Tabelle Ia)

a) ohne lokale Symptome für mindestens eine vorausgehende Woche

b) zwei von diesen mit wiederholten Rezidiven

Tabelle 2

Wirksamkeit von 9-[(Methylamino)-methyl]-9,10-dihydro-9,10-ethanoanthracen-hydrochlorid bei Meerschweinchen mit Dermatitis herpetica verursacht durch HVH 1/Tup.

| Wirkstoff und Dosierung | Anzahl Läsionen a) | Durchschnittliche Fläche der Läsionen am Tag 0 b) | Durchschnittliche Fläche der Läsionen in % der Fläche vom Tag 0 | | | | | gelähmte Tiere |
|---|---|---|---|---|---|---|---|---|
| | | | 2 | 4 | 7 | 9 | 11 | |
| 9-[(Methylamino)-methyl]-9,10-dihydro-9,10-ethanoanthracen-hydrochlorid | | | | | | | | |
| 5,0 mg/kg p.o. | 12 | 86 $mm^2$ | 83** | 66** | 24 | 20 | 15 | 0/6 |
| 1,0 mg/kg p.o. | 12 | 97 $mm^2$ | 75** | 59** | 21 | 13 | 9 | 1/6 |
| 0,1 mg/kg p.o. | 12 | 96 $mm^2$ | 88** | 66** | 20 | 13 | 19 | 1/6 |
| Kontrolle (dest.Wasser) | 12 | 90 $mm^2$ | 122 | 103 | 66 | 45 | 25 | 3/6 |
| | | Anzahl der Läsionen mit Bläschen / totale Anzahl der Läsionen c) | | | | | | |
| 9-[(Methylamino)-methyl]-9,10-dihydro-9,10-ethanoanthracen-hydrochlorid | | | | | | | | |
| 5,0 mg/kg p.o. | 12 | 12/12 | 1/12 §§ | 3/12 §§ | 0/12 | 0/12 | 0/12 | |
| 1,0 mg/kg p.o. | 12 | 12/12 | 6/12 § | 3/12 §§ | 0/12 | 0/12 | 0/12 | |
| 0,2 mg/kg p.o. | 12 | 12/12 | 1/12 §§ | 0/12 §§ | 0/12 | 0/12 | 0/12 | |
| Kontrolle (dest.Wasser) | 12 | 12/12 | 12/12 | 11/12 | 0/12 | 0/12 | 0/12 | |

Behandlung: zweimal täglich während 5 Tagen, beginnend am Tage 0, 3 Tage nach der Infektion.

a) zwei herpetische Läsionen an jedem der 6 Tiere
b) Tag 0 = 100%.
c) in den gleichen Versuchsreihen wie oben.

* p 0,02 } Wilcoxon Rank Order Test
** p 0,005

§ p 0,05 } Fisher Exakt Test
§§ p 0,01

Tabelle 3

Wirksamkeit von 9-[(Methylamino)-methyl]-9,10-dihydro-9,10-ethanoanthracen-hydrochlorid in Meerschweinchen, die intravaginal mit HVH 2/Alabama infiziert wurden.

| Darreichungsform | Dosis mg/kg p.o. | N | Behandlungsbeginn b) nach Infektion a) | Wirkung: % Ueberlebende am Tag 35 c) | Wirkung: Tiere ohne genitale Symptome am Tag 14 | Wirkung: Tiere ohne genitale Symptome am Tag 21 |
|---|---|---|---|---|---|---|
| Tabletten d) (10 mg) | 1,0 | 10 | 72 h | 30 | 30 | 30 |
| | 0,2 | 10 | 72 h | 50 * | 40 ** | 50 ** |
| Placebo | - | 16 | - | 6 | 0 | 0 |
| Sirup (1 mg/ml) | 1,0 | 18 | 48 h | 28 | 22 | 22 |
| | 0,2 | 15 | 48 h | 40 ** | 33 | 33 |
| Placebo | - | 14 | - | 0 | 0 | 0 |

a) Infektion mit $3 \times 10^3$ PFU;

b) Behandlung: 2 x täglich während 5 Tagen

c) Todesfälle traten 10 bis 14 Tage nach Infektion ein

d) gelöst in dest. Wasser

* Signifikant $p < 0,05$ } Fisher Exact Test

** Signifikant $p < 0,01$ } Fisher Exact Test

Tabelle 4

Wirksamkeit von 9-[(Methylamino)-methyl]-9,10-dihydro-9,10-ethano-anthracen-hydrochlorid an Mäusen, die intracerebral mit HVH 1/Tup infiziert wurden.

| Versuchsgruppe | Dosis mg/kg a) p.o. | N | Behandlungszeit b) | Ueberleben der Tiere am Tage 17 in % B/K c) |
|---|---|---|---|---|
| 1 | 1,0 | 14 | - 2 h, 2 x täglich, 5 Tage | 86/7 ** |
| | 0,3 | 14 | - 2 h, 2 x täglich, 5 Tage | 50/7 * |
| | 0,3 | 14 | + 24 h, 2 x täglich, 5 Tage | 57/7 * |
| | 0,3 | 14 | + 48 h, 2 x täglich, 5 Tage | 29/7 |
| 2 | 1,0 | 28 | - 2 h, 2 x täglich, 5 Tage | 32/0 * |
| | 0,3 | 42 | dito | 33/0 ** |
| | 0,1 | 42 | dito | 38/0 ** |
| 3 | 1,0 | 42 | + 24 h, 2 x täglich, 5 Tage | 26/2 * |
| | 0,3 | 42 | dito | 26/2 * |
| | 0,1 | 42 | dito | 50/2 ** |
| 4 | 1,0 | 28 | + 48 h, 2 x täglich, 5 Tage | 11/7 |
| | 0,3 | 28 | dito | 14/7 |
| | 0,1 | 42 | dito | 43/5 * |

a) Lösung in Phosphat-Puffer pH 7.4 (Sörensen) bei Versuchsgruppe 1, Lösung in Aqua dest. bei Versuchsgruppen 2, 3 und 4

b) Beginn der Behandlung vor (-) bzw. nach (+) der Infektion

c) Behandelt/Kontrollen %, $MF_2$-Weibchen mit 16-18 g Gewicht

* Signifikant $p < 0{,}05$ } Fisher Exakt Test
** Signifikant $p < 0{,}01$

Die Verbindungen der Formel I und ihre pharmazeutisch verwenbaren Salze eignen sich daher zur Behandlung von Herpesinfektionen, wie Herpes genitalis, Herpes labialis, Dermatitis und Gingivostomatitis herpetica, Encephalitis herpetica, Herpes zoster und Varicella.

Erfindungsgemäss werden die Verbindungen der Formel I und ihre pharmazeutisch annehmbaren Säureadditionssalze in der zur Behebung oder Linderung von Herpes-Infektionen erforderlichen Menge und insbesondere peroral an behandlungsbedürftige Warmblüter allein oder in Kombination mit anderen Pharmaka verabreicht.

Die perorale Dosierung hängt von der Warmblüter-Spezies, der Applikationsweise, dem Alter und individuellen Zustand des zu behandelnden Warmblüters ab und liegt zwischen ca. 0,01 bis 5,0 mg/kg, vorzugsweise 0,1-2,0 mg/kg, täglich. In der Humanmedizin richtet sich die Dosierung insbesondere auch nach der Intensität der Symptome und ferner danach, ob die oben angegebene Primärwirkung in Kauf genommen werden kann oder gar erwünscht ist. Für erwachsene Personen von normalem Gewicht liegen die Einzeldosen vorzugsweise zwischen 2,5 und 10 mg bei dreimaliger Verabreichung einer Verbindung der Formel I oder ihrer pharmazeutisch annehmbaren Säureadditionssalze, wobei letztere gegebenenfalls entsprechend höher zu dosieren sind, falls sie sich von Säuren mit relativ hohem Molekulargewicht ableiten. Entsprechend erniedrigte bzw. erhöhte Dosen werden an Kinder oder untergewichtige Erwachsene bzw. gegebenenfalls an übergewichtige Erwachsene verabreicht. Je nach der Art der Primärwirkung können im Laufe des Tages unterschiedliche Einzeldosen verabreicht werden, von dem bei höherer Dosierung sedierend wirkenden 9-[(Methylamino)-methyl]-9,10-dihydro-9,10-ethanoanthracen oder dessen pharmazeutisch annehmbaren Salzen, wie dem Hydrochlorid, z.B. morgens und mittags je 2,5 oder 5 mg und am Abend 5 oder 10 mg.

Erfindungsgemäss anwendbare pharmazeutische Zusammensetzungen in Doseneinheitsformen, wie Dragées, Tabletten, Kapseln, Suppositorien oder Ampullen, enthalten als Wirkstoff vorzugsweise 2,5-25 mg, insbesondere 2,5-10 mg, einer Verbindung der Formel I oder vorzugsweise eines pharmazeutisch annehmbaren Säureadditionssalzes einer solchen zusammen mit mindestens einem pharmazeutischen Trägerstoff. Die erfindungsgemäss verwendbaren Wirkstoffe eignen sich auch zur topischen Applikation zusammen mit geeigneten pharmazeutischen Trägermaterialien.

Doseneinheitsformen für die perorale Anwendung enthalten als Wirkstoff vorzugsweise zwischen 1% und 50% einer Verbindung der Formel I oder eines pharmazeutisch annehmbaren Säureadditionssalzes derselben. Zu ihrer Herstellung kombiniert man den Wirkstoff z.B. mit festen, pulverförmigen Trägerstoffen, wie Lactose, Saccharose, Sorbit, Mannit; Stärken, wie Kartoffelstärke, Maisstärke oder Amylopektin, ferner Laminariapulver oder Citruspulpenpulver; Cellulosederivaten oder Gelatine, gegebenenfalls unter Zusatz von Gleitmitteln, wie Magnesium- oder Calciumstearat oder Polyäthylenglykolen, zu Tabletten oder zu Dragée-Kernen. Die Dragée-Kerne überzieht man beispielsweise mit konzentrierten Zuckerlösungen, welche z.B. noch arabischen Gummi, Talk und/oder Titandioxid enthalten können, oder mit einem Lack, der in leichtflüchtigen organischen Lösungsmitteln oder Lösungsmittelgemischen gelöst ist. Diesen Ueberzügen können Farbstoffe zugefügt werden, z.B. zur Kennzeichnung verschiedener Wirkstoffdosen.

Als weitere orale Doseneinheitsformen eignen sich Steckkapseln aus Gelatine sowie weiche, geschlossene Kapseln aus Gelatine und einem Weichmacher, wie Glycerin. Die Steckkapseln enthalten den Wirkstoff vorzugsweise als Granulat, z.B. in Mischung mit Füllstoffen, wie Maisstärke, und/oder Gleitmitteln, wie Talk oder Magnesiumstearat, und gegebenenfalls Stabilisatoren, wie Natriummetabisulfit ($Na_2S_2O_5$) oder Ascorbinsäure. In weichen Kapseln ist der Wirkstoff vorzugsweise

in geeigneten Flüssigkeiten, wie flüssigen Polyäthylenglykolen, gelöst oder suspendiert, wobei ebenfalls Stabilisatoren zugefügt sein können.

Als nicht einzeldosierte perorale Anwendungsformen kommen in üblicher Weise zubereitete Sirupe in Frage, z.B. solche, die den Wirkstoff oder ein pharmazeutisch annehmbares Salz davon in 0,05-0,25%-iger Konzentration dispergiert enthalten, vorzugsweise zusammen mit üblichen Hilfsstoffen wie Polysacchariden oder Polyalkoholen, z.B. Sorbit, Süssstoffen und/oder Aromastoffen und Konservierungsmitteln, z.B. einem Alkalimetallsorbat.

Als erfindungsgemässe Doseneinheitsformen für die rektale Anwendung kommen z.B. Suppositorien in Betracht, welche aus einer Kombination eines Wirkstoffes mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner eignen sich auch Gelatine-Rektalkapseln, welche aus einer Kombination des Wirkstoffes mit einer Grundmasse bestehen. Als Grundmasse eignen sich z.B. flüssige Triglyceride, Polyäthylenglykole oder Paraffinkohlenwasserstoffe.

Ampullen zur parenteralen, insbesondere intramuskulären Verabreichung enthalten vorzugsweise ein wasserlösliches, pharmazeutisch annehmbares Salz des Wirkstoffes z.B. von 9-Methylamino-methyl-9,10-dihydro-9,10-ethanoanthracens in einer Konzentration von vorzugsweise 0,5-5%, gegebenenfalls zusammen mit geeigneten Stabilisierungsmitteln und Puffersubstanzen, in wässriger Lösung.

Weiter kommen zur parenteralen, insbesondere intravenösen Verabreichung Infusionslösungen in Betracht, die z.B. ein wasserlösliches, pharmazeutisch annehmbares Salz des Wirkstoffes z.B. in Sörensen-Phosphatpufferlösung, enthalten, und gegebenenfalls auch unmittelbar vor dem

Gebrauch aus einer Ampullenlösung und dieser Pufferlösung oder einer andern Infusionslösung bereitet werden können.

Als topisch anwendbare pharmazeutische Präparate kommen Crèmen, Salben, Gele, Vaginal-Ovula, Pasten, Schäume, Tinkturen und Lösungen in Betracht, die von etwa 0,1 bis 1% des Wirkstoffs enthalten.

Cremen sind Oel-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Weichparaffine (petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukte davon, wie Polyglycerinfettsäureester oder Polyoxyäthylensorbitan-fettsäureester, ferner Polyoxyäthylen-fettalkoholäther oder -fettsäureester, oder entsprechende ionische Emulgatoren, wie Alkalimetallsalze von Fettalkoholsulfaten, z.B. Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Cremen vermindern, z.B. Polyalkohole, wie Glycerin, Sorbit, Propylenglykol und/oder Polyäthylenglykole, ferner Konservierungsmittel, Riechstoffe, etc.

Salben sind Wasser-in-Oel-Emulsionen, die bis zu 70%, vorzugsweise jedoch von etwa 20% bis etwa 50% Wasser oder wässrige Phase enthalten. Als Fettphase kommen in erster Linie Kohlenwasserstoffe, z.B. Weichparaffine, Paraffinöl und/oder Hartparaffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe; etc.

Fettsalben sind wasserfrei und enthalten als Grundlage insbesondere Kohlenwasserstoffe, z.B. Paraffin, Weichparaffine und/oder flüssige Paraffine, ferner natürliche oder partial-synthetische Fette, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cremen und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Gele sind insbesondere wässrige Lösungen der Wirkstoffe, in denen Gelbildner, vorzugsweise solche aus der Gruppe der Celluloseäther, wie z.B. Methylcellulose, Hydroxyäthylcellulose oder Carboxymethylcellulose, oder der pflanzlichen Hydrokolloide, wie Natriumalginat, Traganth oder Gummi arabicum, dispergiert und ausgequollen sind. Weiter enthalten die Gele vorzugsweise ebenfalls Feuchthaltemittel aus der Gruppe der Polyalkohole, wie Propylenglykol, Glycerin und/oder niedrige Polyäthylenglykole, sowie Netzmittel, z.B. Polyoxyäthylensorbitan-fettsäureester, wie Polyoxyäthylensorbitanmonolaurat. Als weitere Zusatzstoffe können die Gele übliche Konservierungsmittel, z.B. Benzylalkohol, Phenäthylalkohol, Phenoxyäthanol, p-Hydroxybenzoesäureniederalkylester, wie der Methyl- und/oder der Propylester, Sorbinsäure oder deren Alkalimetallsalze, oder organische Quecksilberverbindungen, wie Merthiolat, enthalten.

Tinkturen und Lösungen weisen meistens eine wässerig-äthanolische bzw. wässerige Grundlage auf, der u.a. Polyalkohole, wie z.B. Propylenglykol oder Glycerin und/oder niedrige Polyäthylenglykole, als Feuchthaltemittel zur Herabsetzung der Verdunstung, und gegebenenfalls rückfettende Substanzen, wie Fettsäureester von niedrigen Polyäthylenglykolen, d.h. im wässerig-äthanolischen Gemisch lösliche, lipophile Substanzen, als Ersatz für die der Haut mit dem Aethanol entzogenen Fettsubstanzen, und gegebenenfalls weitere Hilfs- und Zusatzstoffe, neben üblichen, wie den obengenannten, Konservierungsmitteln z.B. auch die bereits erwähnten Polyoxyäthylensorbitan-fettsäureester, wie Polyoxyäthylensorbitan-monolaurat.

Die Herstellung der topisch anwendbaren pharmazeutischen Präparate erfolgt in an sich bekannter Weise, z.B. durch Lösen oder Suspendieren des Wirkstoffs in der Grundlage oder zunächst in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird der feste, vorzugsweise feingemahlene Wirkstoff nach der Dispergierung mit einem Teil der Grundlage vermischt und dann dem Rest der Grundlage beigegeben.

Die erfindungsgemässen Präparate können - neben den üblichen Konservierungsmitteln - auch weitere biologisch, z.B. antiphlogistisch oder antimikrobiell, wie antibakteriell, antifungal oder ebenfalls antiviral wirksame Stoffe, wie z.B. Flumethason, Neomycin, Gentamycin, Milchsäure oder Mikonazol enthalten.

Die erfindungsgemässen topischen Präparate eignen sich insbesondere zur Behandlung von Herpes genitalis, Herpes dermatitis und Herpes labialis. Beispielsweise werden zur Behandlung der beiden ersteren erfindungsgemässe Gele oder Salben, z.B. mittels Tube oder Applikator 2-3 mal täglich, und zur Behandlung von Herpes labialis mehrmals täglich auf die erkrankten Körperstellen aufgetragen bis zum Abklingen der Symptome bzw. bis zur Abheilung.

Erfindungsgemässe wässerige Lösungen können z.B. zum Spülen von erkrankten Körperhöhlen, insbesondere zur Behandlung von Herpes gingivostomatitis, oder zur Behandlung von Herpes keratokonjunktivitis verwendet werden.

Die nachfolgenden Beispiele beschreiben die Herstellung von typischen Applikationsformen, sie sollen jedoch den Umfang der Erfindung in keiner Weise beschränken.

Beispiel 1:

100 g 9-[(Methylamino)-methyl]-9,10-dihydro-9,10-ethanoanthracen-hydrochlorid werden mit 202 g Lactose und 195 g Kartoffelstärke vermischt, die Mischung mit einer alkoholischen Lösung von 10 g Stearinsäure befeuchtet und durch ein Sieb granuliert. Nach dem Trocknen mischt man 200 g Kartoffelstärke, 250 g Talk, 3,0 g Magnesiumstearat und 40 g kolloidales Siliciumdioxid zu und presst die Mischung zu 10'000 Tabletten von je 100 mg Gewicht und 10 mg Wirkstoffgehalt, die gewünschtenfalls mit Teilkerben zur feineren Anpassung der Dosierung versehen sein können.

Beispiel 2:

Aus 50,0 g 9-[(Methylamino)-methyl]-9,10-dihydro-9,10-ethanoanthracen-hydrochlorid, 228,40 g Lactose und der alkoholischen Lösung von 7,5 g Stearinsäure stellt man ein Granulat her, das man nach dem Trocknen mit 56,60 g kolloidalem Siliciumdioxid, 200 g Talk, 20 g Kartoffelstärke und 2,50 g Magnesiumstearat mischt und zu 10'000 Dragée-Kernen presst. Diese werden anschliessend mit einem konzentrierten Sirup aus 417,3 g krist. Saccharose, 6 g Schellack, 10 g arabischem Gummi, 0,2 g Farbstoff und 1,5 g Titandioxid überzogen und getrocknet. Die erhaltenen Dragées wiegen je 120 mg und enthalten je 5 mg Wirkstoff.

Analog kann man unter Verwendung von 25,0 g Wirkstoff und 253,40 g Lactose 10'000 Dragées mit je 2,5 mg Wirkstoff herstellen.

Beispiel 3:

Um 1000 Kapseln mit je 10 mg Wirkstoffgehalt herzustellen, mischt man 10 g 9-[(Methylamino)-methyl]-9,10-dihydro-9,10-ethanoanthracen-hydrochlorid mit 263 g Lactose, befeuchtet die Mischung gleichmässig mit einer wässerigen Lösung von 2 g Gelatine und granuliert sie durch ein geeignetes Sieb (z.B. Sieb III nach Ph. Helv. V). Das Granulat mischt man mit 10 g getrockneter Maisstärke und 15 g Talk und füllt es gleichmässig in 1000 Hartgelatinekapseln der Grösse 1.

Beispiel 4:

Man bereitet eine Suppositoriengrundmasse aus 20 g 9-[(Methylamino)-methyl]-9,10-dihydro-9,10-ethanoanthracen-hydrochlorid und 168,0 g Adeps solidus und giesst damit 100 Suppositorien mit je 20 mg Wirkstoffgehalt.

Beispiel 5:

9-[(Methylamino)-methyl]-9,10-dihydro-9,10-ethanoanthracen-hydrochlorid 0,100 g, Saccharin-Natrium krist. 0,100 g, Sorbit-Lösung 40,000 g, Kaliumsorbat 0,100 g, Citronensäure-Monohydrat 0,500 g, Kirschenaroma 0,100 g, Wasser entmineralisiert bis zu 100 ml.

Herstellung: 9-[(Methylamino)-methyl]-9,10-dihydro-9,10-ethanoanthracen-hydrochlorid, Saccharin-Natrium krist., Kaliumsorbat und Citronensäure-Monohydrat werden in kaltem entmineralisiertem Wasser gelöst. Hierauf werden die Sorbit-Lösung und das Kirschenaroma zugesetzt. Sodann wird das noch fehlende entmineralisierte Wasser ergänzt und kräftig gemischt.

Beispiel 6:

Eine Lösung von 25,0 g 9-[(Methylamino)-methyl]-9,10-dihydro-9,10-ethanoanthracen-hydrochlorid in einem Liter Wasser wird in 1000 Ampullen abgefüllt und sterilisiert. Eine Ampulle enthält eine 2,5%-ige Lösung von 25 mg Wirkstoff.

Beispiel 7:

Zur Herstellung von 5 Liter Gel vermischt man 100 g hochviskose Carboxymethylcellulose mit 500 g Propylenglykol und 3,25 ml Aqua conservans und lässt das Gemisch zu einem homogenen Schleim ausquellen. Dann wird eine Suspension von 50 g 9-[(Methylamino)-methyl]-9,10-di-

hydro-9,10-ethanoanthracen-hydrochlorid in 1 Liter Aqua conservans zugemischt. Schliesslich wird mit Aqua conservans auf 5 Liter ergänzt, sorgfältig gemischt und das erhaltene Gel in Tuben abgefüllt. So erhält man ein Gel mit 1% Wirkstoff.

Unter Aqua conservans wird eine wässerige Lösung von 0,07% p-Hydroxy-benzoesäure-methylester (Methylparaben) und 0,03% p-Hydroxy-benzoesäure-propylester (Propylparaben) verstanden.

Patentansprüche

1. Verfahren zur Behandlung von Herpes-Infektionen, dadurch gekennzeichnet, dass man eine Verbindung der Formel

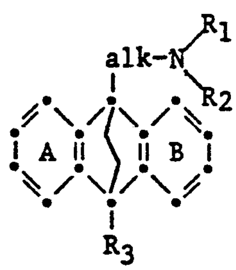

I

worin alk für Alkylen mit 1-4 Kohlenstoffatomen steht, $R_1$ und $R_2$ unabhängig voneinander Wasserstoff, Alkyl mit 1-4 Kohlenstoffatomen bedeuten oder zusammen für einen Alkylenrest mit 4 bis 6 Kohlenstoffatomen stehen, $R_3$ Wasserstoff, Methyl oder Chlor bedeuten, und die Ringe A und B unabhängig voneinander unsubstituiert oder durch Chlor substituiert sein können oder eines ihrer pharmazeutisch annehmbaren Säureadditionssalze verwendet.

2. Verfahren nach Anspruch 1, dadurch gekennzeichnet, dass man als Verbindung der Formel I eine solche verwendet, in der alk für Methylen oder Trimethylen steht, $R_1$ und $R_2$ unabhängig voneinander Wasserstoff oder Methyl, $R_3$ Wasserstoff bedeuten und die Ringe A und B unsubstituiert sind, oder ein pharmazeutisch annehmbares Salz davon verwendet.

3. Verfahren nach Anspruch 1, dadurch gekennzeichnet, dass man als Verbindung der Formel I 9-(γ-Methylaminopropyl)-9,10-dihydro-9,10-ethanoanthracen, oder ein pharmazeutisch annehmbares Salz davon verwendet.

4. Verfahren nach Anspruch 1, dadurch gekennzeichnet, dass man als Verbindung der Formel I 9-[(Methylamino)-methyl]-9,10-dihydro-9,10-ethanoanthracen, oder ein pharmazeutisch annehmbares Salz davon verwendet.

5. Verfahren nach Anspruch 3 oder 4, dadurch gekennzeichnet, dass man als Salz das Hydrochlorid verwendet.

6. Verfahren nach einem der Ansprüche 1 bis 5, dadurch gekennzeichnet, dass man eine Verbindung der Formel I oder ein pharmazeutisch annehmbares Säureadditionssalz einer solchen in oralen Tagesdosierungen von 0,01 bis 5,0 mg/kg an Warmblüter verabreicht.

7. Verfahren nach einem der Ansprüche 1 bis 6, dadurch gekennzeichnet, dass die Tagesdosis 0,1 bis 2,0 mg/kg beträgt.

8. Verwendung der in den Ansprüchen 1 bis 4 genannten Verbindungen oder ihrer pharmazeutisch annehmbaren Säureadditionssalze zur Behandlung von Herpesinfektionen.

9. Antiherpesmittel, enthaltend mindestens eine der in den Ansprüchen 1 bis 4 genannten Verbindungen oder eines ihrer pharmazeutisch annehmbaren Säureadditionssalze.

10. Orale oder parenterale Antiherpesmittel, enthaltend mindestens eine der in den Ansprüchen 1 bis 4 genannten Verbindungen oder eines ihrer pharmazeutisch annehmbaren Säureadditionssalze.

11. Orale oder parenterale Antiherpesmittel, enthaltend 2,5-10 mg einer der in den Ansprüchen 1 bis 4 genannten Verbindungen oder eines ihrer pharmazeutisch annehmbaren Säureadditionssalze.

12. Topische Arzneimittel, enthaltend mindestens eine der in den Ansprüchen 1 bis 4 genannten Verbindungen oder eines ihrer pharmazeutisch annehmbaren Säureadditionssalze.

13. Topische Arzneimittel nach Anspruch 12, enthaltend eine der in den Ansprüchen 1 bis 4 genannten Verbindungen oder eines ihrer pharmazeutisch annehmbaren Säureadditionssalze in einer Konzentration von 0,05 bis 1%.

Europäisches Patentamt

**EUROPÄISCHER TEILRECHERCHENBERICHT,** der nach Regel 45 des Europäischen Patent-übereinkommens für das weitere Verfahren als europäischer Recherchenbericht gilt

Nummer der Anmeldung
EP 80 81 0247

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| DX | US - A - 3 399 201 (P. SCHMIDT)<br>* Ansprüche; Beispiele 1,2; Spalte 2, Zeilen 29-36 *<br>---- | |

KLASSIFIKATION DER ANMELDUNG (Int. Cl.³)
A 61 K 31/135/
C 07 C 87/458

RECHERCHIERTE SACHGEBIETE (Int. Cl.³)
A 61 K 31/135

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche: 8-13
Unvollständig recherchierte Patentansprüche
Nicht recherchierte Patentansprüche 1-7
Grund für die Beschränkung der Recherche: Verfahren zur chirurgischen oder therapeutischen Behandlung des menschlichen oder tierischen Körpers (Siehe Art. 52(4) des Europäischen Patentübereinkommens).

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 31-10-1980 | NUYTS |